# EUROPÄISCHE PATENTSCHRIFT

(11) **EP 1 100 456 B1**
(45) Veröffentlichungstag und Bekanntmachung des Hinweises auf die Patenterteilung: **25.09.2002**
(21) Anmeldenummer: 99938318.5
(22) Anmeldetag: 22.07.1999
(51) Int. Cl.: A61K 7/48

(54) **VERWENDUNG VON ECTOIN ODER ECTOIN-DERIVATEN IN FORMULIERUNGEN**
USE OF ECTOINE OR ECTOINE DERIVATIVES IN FORMULATIONS
UTILISATION D'ECTOINE OU DE DERIVES D'ECTOINE DANS DES FORMULATIONS

(30) Priorität: 01.08.1998 DE 19834816
(43) Veröffentlichungstag der Anmeldung: 23.05.2001
(73) Patentinhaber: MERCK PATENT GmbH, 64293 Darmstadt (DE)
(72) Erfinder: BÜNGER, Joachim, D-64823 Gross-Umstadt (DE); DRILLER, Hans-Jürgen, D-64823 Gross-Umstadt (DE)
(86) Internationale Anmeldenummer: EP9905237
(87) Internationale Veröffentlichungsnummer: WO00007558

(56) Entgegenhaltungen:
- EP-A- 0 275 719
- WO-A-98/13020
- US-A- 5 738 858

## Beschreibung

Die Erfindung betrifft die Verwendung von einer oder mehreren Verbindungen ausgewählt aus den Verbindungen der Formeln la und lb 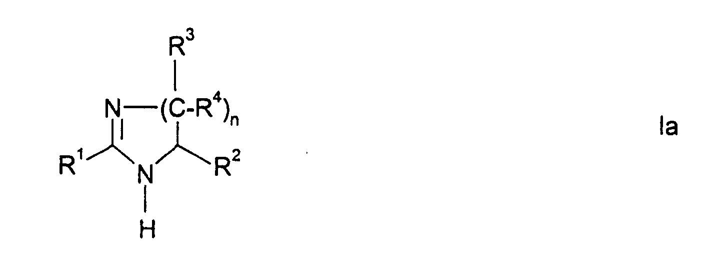 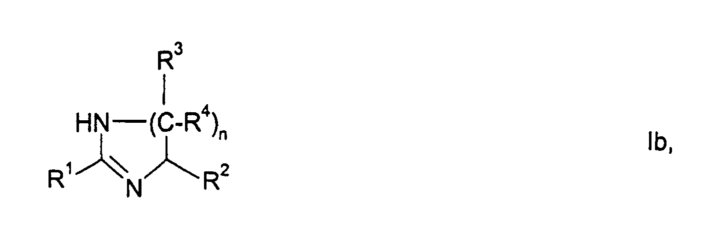 den physiologisch verträglichen Salzen der Verbindungen der Formeln la und Ib, und den stereoisomeren Formen der Verbindungen der Formeln la und Ib, wobei
- R¹: H oder Alkyl,
- R²: H, COOH, COO-Alkyl oder CO-NH-R⁵,
- R³ und R⁴: jeweils unabhängig voneinander H oder OH,
- n: 1, 2 oder 3,
- Alkyl: einen Alkylrest mit 1 bis 4 Kohlenstoffatomen, und
- R⁵: H, Alkyl, einen Aminosäurerest, Dipeptidrest oder Tripeptidrest
bedeuten, zur Herstellung einer Formulierung, insbesondere einer kosmetischen Formulierung, zum Schutz und zur Stabilisierung der Nukleinsäuren von menschlichen Hautzellen.

Die Erfindung betrifft insbesondere die Verwendung von einer oder mehreren Verbindungen ausgewählt aus den Verbindungen der Formeln la und lb, den physiologisch verträglichen Salzen der Verbindungen der Formeln la und lb und den stereoisomeren Formen der Verbindungen der Formeln la und lb zur Herstellung einer Formulierung zum Schutz und zur Stabilisierung der Nukleinsäuren von menschlichen Hautzellen vor physikalischen, chemischen und biologischen Einflüssen wie z.B. vor Strahlung (UV-, VIS- und IR-Strahlung), insbesondere UV-Strahlung, vor denaturierenden Substanzen, vor Enzymen, insbesondere Endonukleasen und Restriktionsenzymen und vor Viren, insbesondere Herpes-Viren.

Die Erfindung betrifft weiterhin die Verwendung von einer oder mehreren Verbindungen ausgewählt aus den Verbindungen der Formeln la und Ib, den physiologisch verträglichen Salzen der Verbindungen der Formeln la und lb und den stereoisomeren Formen der Verbindungen der Formeln la und lb zusammen mit einem oder mehreren UV-Filtern zur Herstellung einer Formulierung zum Schutz und zur Stabilisierung der Nukleinsäuren von menschlichen Hautzellen vor UV-Strahlung, insbesondere vor UV-A-Strahlung.

Die Nukleinsäuren, Desoxyribonukleinsäure (DNS bzw. DNA) und Ribonukleinsäuren (RNS bzw. RNA), treten in allen Zellen des menschlichen Körpers auf. Sie spielen eine entscheidende Rolle bei der Vererbung und regeln den Aufbau der Proteine, die für das Leben und die Funktion jeder Zelle notwendig sind. Die Desoxyribonukleinsäure trägt die Information für die Proteinsynthese, während die Ribonukleinsäuren an der Weiterleitung dieser Informationen in die Proteinsynthese beteiligt sind. Schädigungen der Nukleinsäuren von menschlichen Hautzellen, insbesondere der Desoxyribonukleinsäure, können zu chronischen Hautveränderungen führen.

Die Haut ist als Grenzschicht und Oberfläche des menschlichen Körpers einer Vielzahl externer Streßfaktoren ausgesetzt. Die Human-Haut ist ein Organ, das mit verschiedenartig spezialisierten Zelltypen - den Keratinozyten, Melanozyten, Langerhans-Zellen, Merkel-Zellen und eingelagerten Sinneszellen - den Körper vor äußeren Einflüssen schützt. Hierbei ist zwischen äußeren physikalischen, chemischen und biologischen Einflüssen auf die menschliche Haut zu unterscheiden. Zu den äußeren physikalischen Einflüssen sind thermische und mechanische Einflüsse sowie die Einwirkung von Strahlung, z.B. UV-, VIS- und IR-Strahlung, zu zählen. Unter den äußeren chemischen Einflüssen sind insbesondere die Einwirkung von Toxinen, Allergenen und Substanzen, die an die Desoxyribonukleinsäure anbinden, zu verstehen. Die äußeren biologischen Einflüsse umfassen die Einwirkung fremder Organismen und deren Stoffwechselprodukte.

Das Sonnenlicht hat sowohl positive als auch negative Wirkungen auf die menschliche Haut und den gesamten Organismus. Bei geeigneter Dosierung steigert die Sonnenbestrahlung das Wohlbefinden und die Leistungsfähigkeit des Organismus. Die Vitamin-D-Synthese wird stimuliert, und schließlich entwickelt sich als Folge der Bestrahlung die begehrte Bräune oder Pigmentierung der Haut. Die Pigmentierung ist Teil des Eigenschutzes der Haut, der auf einer Vielzahl von Mechanismen beruht. Im Zusammenhang mit dem Eigenschutz der Haut sind neben der Pigmentierung insbesondere die Verdickung der Homschicht (Lichtschwiele), das Dark-Repair-System (enzymatische DNS-Reparatur), die Redoxsysteme zur Kontrolle von radikalischen Reaktionen und die Synthese von Urocaninsäure von Bedeutung (P. Finkel, "Lichtschutzmittel" in W. Umbach, Kosmetik, 2. Auflage, 1995, 147-163, Georg Thieme Verlag, Stuttgart).

Eine übermäßige Sonnenbestrahlung führt sowohl zu akuten Hautschäden wie beispielsweise Sonnenbrand als auch zu chronischen Veränderungen wie z.B. Hautalterung oder Hautkrebs. Der Sonnenbrand (Erythema solare) entwickelt sich überwiegend als Folge der UV-B-Bestrahlung. Die UV-A-Strahlung hat dagegen einen vergleichsweise geringen Einfluß auf seine Entstehung. Der Sonnenbrand kann von einer leichten Rötung bis hin zu einer starken Verbrennung mit Blasenbildung auftreten. Da diese Folgen frühestens 4-6 h nach der Bestrahlung auftreten, ist es für Gegenmaßnahmen zu spät. Sonnenbrand ist ein Beleg für akute Hautschädigungen, die für chronische Veränderungen der Haut von Relevanz sein können. Mehrere Sonnenbrände - ganz besonders in der Kindheit - erhöhen deutlich das Hautkrebsrisiko. Ursachen hierfür sind Schädigungen, insbesondere der Nukleinsäuren von menschlichen Hautzellen und eine fehlerhafte Reparatur der geschädigten Desoxyribonukleinsäure im Zellkern sowie wahrscheinlich die immunsupressive Wirkung der UV-Strahlung, d.h. die Schwächung der Immunreaktion durch UV-Bestrahlung. Die übermäßige UV-A- und UV-B-Exposition trägt zur Hautalterung bzw. Lichtalterung bei, z.B. in Form von strukturellen Veränderungen des Bindegewebes (aktinische Elastose). Die übermäßige UV-B-Exposition ist die wesentliche Ursache für chronische Hautveränderungen.

Aufgrund eines veränderten Freizeitverhaltens, wie z.B. ausgiebiges Sonnenbaden oder Fernreisen in Länder mit einer starken Sonneneinstrahlung, sind die Gefahren einer UV-Schädigung der Hautzellen in den letzten Jahren stark angestiegen, was sich wiederum in einer Erhöhung des Hautkrebsrisikos niederschlägt (P. Finkel, "Lichtschutzmittel" in W. Umbach, Kosmetik, 2. Auflage, 1995, 147-163, Georg Thieme Verlag, Stuttgart). Ein besonderes Gefahrenpotential stellen Fernreisen in Länder mit starker Sonneneinstrahlung im Winter dar. Die Winterhaut, z.B. von Nordeuropäern, ist wenig pigmentiert und nicht gegen eine starke Sonnenexposition in tropischen, Äquator-nahen Regionen mit einer langen Sonnenscheindauer pro Tag geschützt. Zusätzlich ist das Hautkrebsrisiko in jüngerer Zeit durch eine zunehmende UV-Strahlung an der Erdoberfläche, ausgelöst durch die Abnahme der Ozonschicht, und durch eine höhere Lebenserwartung der Menschheit deutlich angestiegen.

Es bestand daher die Aufgabe, Formulierungen, insbesondere kosmetische Formulierungen, zur Verfügung zu stellen, deren Anwendung die obengenannten Hautprobleme beseitigen oder zumindest mindern und insbesondere zum Schutz und zur Stabilisierung der Nukleinsäuren von menschlichen Hautzellen geeignet sind.

Insbesondere bestand die Aufgabe, Formulierungen zur Verfügung zu stellen, die zum Schutz und zur Stabilisierung der Nukleinsäuren von menschlichen Hautzellen vor physikalischen, chemischen und biologischen Einflüssen wie z.B. vor Strahlung (UV-, VIS- und IR-Strahlung), insbesondere UV-Strahlung, vor denaturierenden Substanzen, vor Enzymen, insbesondere Endonukleasen und Restriktionsenzymen und vor Viren, insbesondere Herpes-Viren, geeignet sind.

Überraschend wurde nun gefunden, daß diese Aufgabe durch die Verwendung von einer oder mehreren Verbindungen ausgewählt aus den Verbindungen der Formeln la und lb 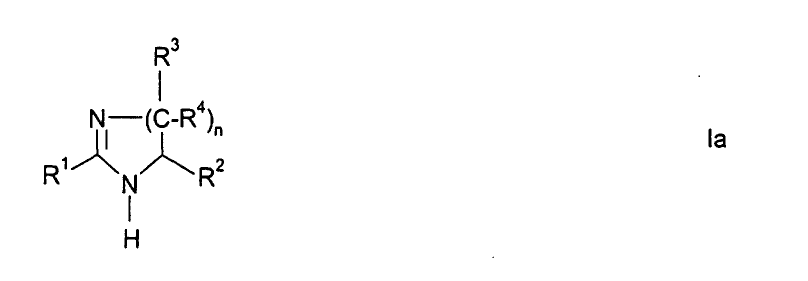 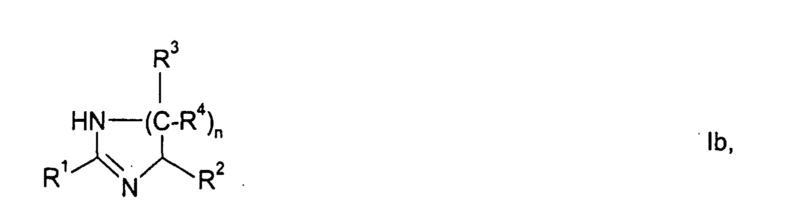 den physiologisch verträglichen Salzen der Verbindungen der Formeln la und lb, und den stereoisomeren Formen der Verbindungen der Formeln la und lb, wobei
- R¹: H oder Alkyl,
- R²: H, COOH, COO-Alkyl oder CO-NH-R⁵,
- R³ und R⁴: jeweils unabhängig voneinander H oder OH,
- n: 1, 2 oder 3,
- Alkyl: einen Alkylrest mit 1 bis 4 Kohlenstoffatomen, und
- R⁵: H, Alkyl, einen Aminosäurerest, Dipeptidrest oder Tripeptidrest
bedeuten,
in Formulierungen, insbesondere in kosmetischen Formulierungen, gelöst wird.

Es wurde zudem gefunden, daß die Verwendung von einer oder mehreren Verbindungen ausgewählt aus den Verbindungen der Formeln la und Ib, den physiologisch verträglichen Salzen der Verbindungen der Formeln la und lb und den stereoisomeren Formen der Verbindungen der Formeln la und lb zusammen mit einem oder mehreren UV-Filtern zur Herstellung einer Formulierung zum Schutz und zur Stabilisierung der Nukleinsäuren von menschlichen Hautzellen vor UV-Strahlung besonders geeignet ist.

Im Rahmen der vorliegenden Erfindung werden alle vor- und nachstehenden Verbindungen ausgewählt aus den Verbindungen der Formeln la und Ib, den physiologisch verträglichen Salzen der Verbindungen der Formeln la und lb, und den stereoisbmeren Formen der Verbindungen der Formeln la und lb als "Ectoin oder Ectoin-Derivate" bezeichnet.

Die Erfindung betrifft weiterhin Formulierungen, insbesondere kosmetische Formulierungen, enthaltend
a) eine oder mehrere Verbindungen ausgewählt aus den Verbindungen der Formeln la und lb 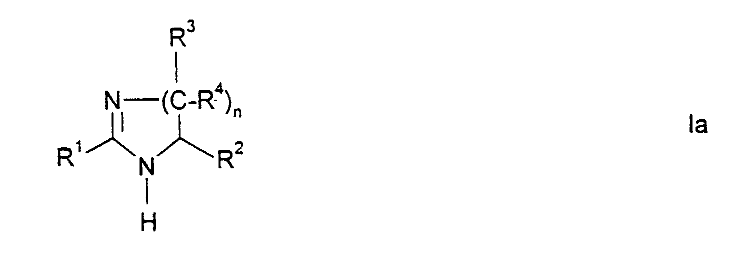 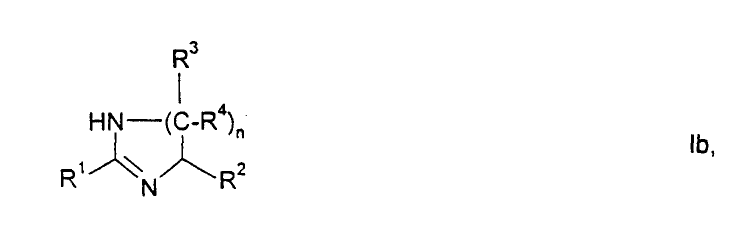 den physiologisch verträglichen Salzen der Verbindungen der Formeln la und Ib, und den stereoisomeren Formen der Verbindungen der Formeln la und Ib, wobei
   - R¹: H oder Alkyl,
   - R²: H, COOH, COO-Alkyl oder CO-NH-R⁵,
   - R³ und R⁴: jeweils unabhängig voneinander H oder OH,
   - n: 1, 2 oder 3,
   - Alkyl: einen Alkylrest mit 1 bis 4 Kohlenstoffatomen, und
   - R⁵: H, Alkyl, einen Aminosäurerest, Dipeptidrest oder Tripeptidrest
   bedeuten
   und
b) einen oder mehrere UV-Filter.

Formulierungen, die Ectoin oder Ectoin-Derivate enthalten, schützen insbesondere Desoxyribonukleinsäure von menschlichen Hautzellen gegen physikalischen Streß, insbesondere gegen Strahlung wie z.B. UV-, VIS oder IR-Strahlung, und gegen chemischen Streß. Insbesondere Formulierungen enthaltend Ectoin oder Ectoin-Derivate und Sonnenschutzfilter, insbesondere UV-Filter, bieten einen hervorragenden Schutz vor UV-induzierten Schäden der Desoxyribonukleinsäure von Hautzellen.

Nach UV-Bestrahlung treten in der menschlichen Epidermis einzeln gelagerte Keratinozyten mit besonderer Charakteristik auf, die sogenannten "Sunburn cells" (SBCs). Die Bildung von "Sunburn cells" wird als ein Beispiel von programmiertem Zelltod, der Apoptose, beschrieben. Die Entstehung von "Sunburn cells" mit ihrem charakterischen pyknotischen Kern und dem eosinophilen Zytoplasma läßt sich in der menschlichen Epidermis bereits nach einer moderaten Bestrahlung mit UVC oder UVB oder UVA in Kombination mit Psoralen nachweisen. Das Schicksal einer SBC, einem Keratinozyten welcher eine abnormale und viel zu frühe Verhomung zeigt, ist bis heute unbekannt. Welches photochemische Ereignis zur Bildung von SBCs führt, ist ebenfalls unbekannt. Es gibt jedoch Evidenzen dafür, daß UV-vermittelte DNA-Schäden bei diesem Prozeß eine wichtige Rolle spielen. Es wird z.B. vermutet, daß die Anwesenheit von SBCs ein möglicher Indikator und Hinweis auf ein gegebenes photokarzinogenes Potential sein könne. SBCs zeigen im Eosin angefärbten Zytoplasma starke Vacuolenbildung, sowie eine Schrumpfung mit stark kondensiertem Zellkern. Sie lösen sich aus dem Zellverband und zeigen eine überstürzte, vorzeitige Verhornung. Sie sind histologisch in den Schnittpräparaten leicht nachweisbar und im Verhältnis zur Zahl der Basalzellen oder zur Gesamtzahl der Keratinozyten quantitativ auswertbar. Das Aktionsspektrum der SBC-Bildung deckt sich mit dem des Erythems. Eine Photo-Augmentation durch UVA und Psoralene (PUVA) führt ebenfalls zu einer dosisabhängigen SBC-Bildung [A.R. Young, 'The sunburn cell', Photodermatology 4 (1987) 127-134; G. Kindl et al., 'Die Wirkung der Sonnenstrahlen' in: Licht und Haut, Verlag Govi, Frankfurt am Main (1993) 51-69; H. lizuka, 'Effects of UVB irradiation on epidermal adenylate cyclase responses in vitro: its relation to Sunburn cell formation', Arch. Dermatol. Res. 280 (1988) 163-167].

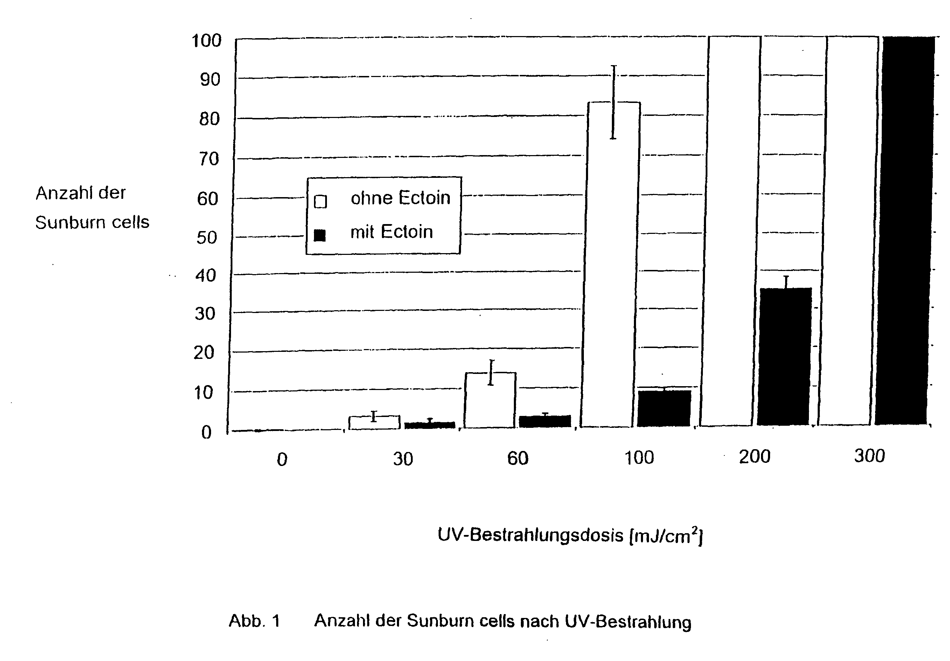

Im Rahmen der vorliegenden Erfindung wurde Ectoin bezüglich seines möglichen Potentials zur Reduktion der UV-vermittelten Bildung von SBCs untersucht. Um dieses Potential eines Wirkstoffkonzeptes korrekt analysieren zu können, ist es erforderlich, das Erscheinen und die Bildung von SBCs in einem physiologischen Modell zu studieren, welches der in vivo Situation möglichst am nächsten kommt. Diese Voraussetzung ist am ehesten beim Einsatz organotypischer Hautmodelle für solche Studien gegeben. Es konnte gezeigt werden, daß zwischen der Bildungsrate von SBCs und der verabreichten UV-Dosis eine Dosiswirkungsbeziehung gegeben ist. Diese Dosiswirkungsbeziehung konnte durch eine Vorbehandlung mit Ectoin signifikant in höhere UV-Dosisbereiche verschoben werden (s. Abb. 1).

Topisch applizierte Formulierungen, die Ectoin oder Ectoin-Derivate enthalten, schützen zudem Nukleinsäuren vor denaturierenden Substanzen und Enzymen wie z.B. Endonukleasen und Restriktionsenzymen. Ectoin oder Ectoin-Derivate können auch in Formulierungen gegen Viren, insbesondere Herpes-Viren, verwendet werden.

Bei Ectoin und den Ectoin-Derivaten handelt es sich um niedermolekulare, cyclische Aminosäurederivate, die aus verschiedenen halophilen Mikroorganismen gewonnen werden können. Sowohl Ectoin als auch Hydroxyectoin besitzen den Vorteil, daß sie nicht mit dem Zellstoffwechsel reagieren.

Die Verbindungen ausgewählt aus den Verbindungen der Formeln la und Ib, den physiologisch verträglichen Salzen der Verbindungen der Formeln la und lb und den stereoisomeren Formen der Verbindungen der Formeln la und lb können in den Zubereitungen als optische lsomere, Diastereomere, Racemate, Zwitterionen, Kationen oder als Gemisch derselben vorliegen. Unter den Verbindungen ausgewählt aus den Verbindungen der Formeln la und Ib, den physiologisch verträglichen Salzen der Verbindungen der Formeln la und lb und den stereoisomeren Formen der Verbindungen der Formeln la und Ib, sind diejenigen Verbindungen bevorzugt, worin R¹ H oder CH₃, R² H oder COOH, R³ und R⁴ jeweils unabhängig voneinander H oder OH und n 2 bedeuten. Unter den Verbindungen ausgewählt aus den Verbindungen der Formeln la und lb, den physiologisch verträglichen Salzen der Verbindungen der Formeln la und lb und den stereoisomeren Formen der Verbindungen der Formeln la und lb sind die Verbindungen (S)-1,4,5,6-Tetrahydro-2-methyl-4-pyrimidincarbonsäure (Ectoin) und (S,S)-1,4,5,6-Tetrahydro-5-hydroxy-2-methyl-4-pyrimidincarbonsäure (Hydroxyectoin) insbesondere bevorzugt.

Unter dem Begriff "Aminosäure" werden die stereoisomeren Formen, z.B. D- und L-Formen, folgender Verbindungen verstanden: Alanin, β-Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin, γ-Aminobutyrat, Nε-Acetyllysin, Nδ-Acetylornitnin, Nγ-Acetyldiaminobutyrat und Nα-Acetyldiaminobutyrat. L-Aminosäuren sind bevorzugt.

Aminosäurereste leiten sich von den entsprechenden Aminosäuren ab.

Die Reste folgender Aminosäuren sind bevorzugt: Alanin, β-Alanin, Asparagin, Asparaginsäure, Glutamin, Glutaminsäure, Glycin, Serin, Threonin, Valin, γ-Aminobutyrat, Nε-Acetyllysin, Nδ-Acetylornithin, Nγ-Acetyldiaminobutyrat und Nα-Acetyldiaminobutyrat.

Die Di- und Tripeptidreste sind ihrer chemischen Natur nach Säureamide und zerfallen bei der Hydrolyse in 2 oder 3 Aminosäuren. Die Aminosäuren in den Di- und Tripeptidresten sind durch Amidbindungen miteinander verbunden. Bevorzugte Di- und Tripetidreste sind aus den bevorzugten Aminosäuren aufgebaut.

Die Alkylgruppen umfassen die Methylgruppe CH₃, die Ethylgruppe C₂H₅, die Propylgruppen CH₂CH₂CH₃ und CH(CH₃)₂ sowie die Butylgruppen CH₂CH₂CH₂CH₃, H₃CCHCH₂CH₃, CH₂CH(CH₃)₂ und C(CH₃)₃. Die bevorzugte Alkylgruppe ist die Methylgruppe.

Bevorzugte physiologisch verträgliche Salze der Verbindungen der Formeln la und lb sind beispielsweise Alkali-, Erdalkali- oder Ammoniumsalze, wie Na-, K-, Mg- oder Ca-Salze, sowie Salze abgeleitet von den organischen Basen Triethylamin oder Tris-(2-hydroxy-ethyl)-amin. Weitere bevorzugte physiologisch verträgliche Salze der Verbindungen der Formeln la und lb ergeben sich durch Umsetzung mit anorganischen Säuren wie Salzsäure, Schwefelsäure und Phosphorsäure oder mit organischen Carbon- oder Sulfonsäuren wie Essigsäure, Citronensäure, Benzoesäure, Maleinsäure, Fumarsäure, Weinsäure und p-Toluolsulfonsäure.

Verbindungen der Formeln la und lb, in denen basische und saure Gruppen wie Carboxyl- oder Aminogruppen in gleicher Zahl vorliegen, bilden innere Salze.

Die Herstellung der Verbindungen der Formel la und lb ist in der Literatur beschrieben (DE 43 42 560). (S)-1,4,5,6-Tetrahydro-2-methyl-4-pyrimidincarbonsäure oder (S,S)-1,4,5,6-Tetrahydro-5-hydroxy-2-methyl-4-pyrimidincarbonsäure können auch mikrobiologisch gewonnen werden (Severin et al., J. Gen. Microb. 138 (1992) 1629-1638).

Für die erfindungsgemäße Verwendung von einer oder mehreren Verbindungen ausgewählt aus den Formeln la und lb, den physiologisch verträglichen Salzen der Verbindungen der Formeln la und lb und den stereoisomeren Formen der Verbindungen der Formeln la und lb zusammen mit einem oder mehreren UV-Filtern und für die erfindungsgemäße Formulierung enthaltend einen oder mehrere UV-Filter sind sowohl organische als auch anorganische UV-Filter geeignet.

Geeignete organische UV-Filter sind z.B. Benzoyl- oder Dibenzoylmethanderivate, Methoxyzimtsäureester, Salicylatderivate, Benzylidencampherderivate, Octocrylen, Benzophenone, Phenylbenzimidazol-5-sulfonsäure, 4-Aminobenzoesäure, Octyl-Triazon und Octyl-dimethyl PABA.

Geeignete anorganische UV-Filter sind z.B. Titandioxid und Zinkoxid.

Nach der vorliegenden Erfindung führt die Verwendung von einer oder mehreren Verbindungen ausgewählt aus den Verbindungen der Formeln la und lb, den physiologisch verträglichen Salzen der Verbindungen der Formeln la und lb und den stereoisomeren Formen der Verbindungen der Formeln la und lb gegebenenfalls zusammen mit einem oder mehreren UV-Filtern zur Herstellung einer Formulierung vorzugsweise zum Schutz und zur Stabilisierung der Nukleinsäuren der Zellen der Epidermis, insbesondere der Keratinozyten, Melanozyten, Langerhans-Zellen und Merkel-Zellen.

Die Herstellung der Formulierung erfolgt, indem eine oder mehrere Verbindungen ausgewählt aus den Verbindungen der Formeln la und lb, den physiologisch verträglichen Salzen der Verbindungen der Formeln la und lb und den stereoisomeren Formen der Verbindungen der Formeln la und lb gegebenenfalls zusammen mit einem oder mehreren UV-Filtern und gegebenenfalls mit Hilfs- und/oder Trägerstoffen in eine geeignete Formulierungsform gebracht werden. Die Hilfs- und Trägerstoffe stammen aus der Gruppe der Trägermittel, Konservierungsstoffe und anderer üblicher Hilfsstoffe.

Die Formulierungen auf der Grundlage einer oder mehrerer Verbindungen ausgewählt aus den Verbindungen der Formeln la und Ib, den physiologisch verträglichen Salzen der Verbindungen der Formeln la und lb und den stereoisomeren Formen der Verbindungen der Formeln la und lb und gegebenenfalls zusätzlich auf der Grundlage von einem oder mehreren UV-Filtern werden äußerlich angewendet.

Als Anwendungsform seien z.B. genannt: Lösungen, Suspensionen, Emulsionen, Pasten, Salben, Gele, Cremes, Lotionen, Puder, Seifen, tensidhaltige Reinigungspräparate, Öle und Sprays. Zusätzlich zu einer oder mehreren Verbindungen ausgewählt aus den Verbindungen der Formeln la und Ib, den physiologisch verträglichen Salzen der Verbindungen der Formeln la und lb und den stereoisomeren Formen der Verbindungen der Formeln la und lb und gegebenenfalls zusätzlich zu einem oder mehreren UV-Filtern werden der Formulierung beliebige übliche Trägerstoffe, Hilfsstoffe und gegebenenfalls weitere Wirkstoffe zugesetzt.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können neben einer oder mehreren Verbindungen ausgewählt aus den Verbindungen der Formeln la und lb, den physiologisch verträglichen Salzen der Verbindungen der Formeln la und lb und den stereoisomeren Formen der Verbindungen der Formeln la und lb und gegebenenfalls neben einem oder mehreren UV-Filtern die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können neben einer oder mehreren Verbindungen ausgewählt aus den Verbindungen der Formeln la und lb, den physiologisch verträglichen Salzen der Verbindungen der Formeln la und lb und den stereoisomeren Formen der Verbindungen der Formeln la und lb und gegebenenfalls neben einem oder mehreren UV-Filtern die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können neben einer oder mehreren Verbindungen ausgewählt aus den Verbindungen der Formeln la und lb, den physiologisch verträglichen Salzen der Verbindungen der Formeln la und lb und den stereoisomeren Formen der Verbindungen der Formeln la und lb und gegebenenfalls neben einem oder mehreren UV-Filtern die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können neben einer oder mehreren Verbindungen ausgewählt aus den Verbindungen der Formeln la und lb, den physiologisch verträglichen Salzen der Verbindungen der Formeln la und lb und den stereoisomeren Formen der Verbindungen der Formeln la und lb und gegebenenfalls neben einem oder mehreren UV-Filtern die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können neben einer oder mehreren Verbindungen ausgewählt aus den Verbindungen der Formeln la und lb, den physiologisch verträglichen Salzen der Verbindungen der Formeln la und lb und den stereoisomeren Formen der Verbindungen der Formein la und lb und gegebenenfalls neben einem oder mehreren UV-Filtern die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können neben einer oder mehreren Verbindungen ausgewählt aus den Verbindungen der Formeln la und lb, den physiologisch verträglichen Salzen der Verbindungen der Formeln la und lb und den stereoisomeren Formen der Verbindungen der Formeln la und lb und gegebenenfalls neben einem oder mehreren UV-Filtern die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können neben einer oder mehrerer Verbindungen ausgewählt aus den Verbindungen der Formeln la und Ib, den physiologisch verträglichen Salzen der Verbindungen der Formeln la und lb und den stereoisomeren Formen der Verbindungen der Formeln la und lb und gegebenenfalls neben einem oder mehreren UV-Filtern die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typisch kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Der Anteil der Verbindungen ausgewählt aus den Verbindungen der Formeln la und lb, den physiologisch verträglichen Salzen der Verbindungen der Formeln la und lb und den stereoisomeren Formen der Verbindungen der Formeln la und lb in der Formulierung beträgt vorzugsweise von 0,0001 bis 50 Gew.%, besonders bevorzugt von 0,001 bis 10 Gew.% bezogen auf die gesamte Formulierung.

Wenn UV-Filter in der Formulierung enthalten sind, beträgt ihr Anteil vorzugsweise von 0,001 bis 50 Gew.-%, besonders bevorzugt von 0,01 Gew.-% bis 10 Gew.-% bezogen auf die gesamte Formulierung.

DNS-Schäden von Hautzellen können beispielsweise durch etablierte und validierte Tests bestimmt werden, z.B. mittels "Comet-Assay".

Der Nachweis des Schutzes der DNS durch Ectoin oder seine Derivate vor Endonukleasen oder Restriktionsenzymen kann z.B. in vitro erfolgen. Hierzu wird gezielt eine Fragmentierung der DNS durch die genannten Enzyme herbeigeführt. Der Fragmentierungsgrad wird anschließend mittels Elektrophorese bestimmt. Ein Vergleich der Ergebnisse für Fragmentierungsversuche, die einerseits in Gegenwart von Ectoin und anderseits ohne Ectoin durchgeführt wurden, liefert einen Nachweis des Schutzes der DNS durch Ectoin oder Ectoin-Derivaten vor Endonukleasen und Restriktionsenzymen.

Alle Verbindungen oder Komponenten, die in den Formulierungen verwendet werden können, sind entweder bekannt und käuflich erwerbbar oder können nach bekannten Methoden synthetisiert werden.

Die folgenden Beispiele dienen zur Verdeutlichung der Erfindung und sind keinesfalls als Limitierung aufzufassen. Alle %-Angaben sind Gewichtsprozent.

### Beispiel 1

Aus folgenden Komponenten wird ein erfindungsgemäßes Hautpflegegel (O/W) enthaltend Ectoin hergestellt:

| | | | | Gew.-% |
|---|---|---|---|---|
| A | Mandelöl | | (2) | 8.0 |
| | Eutanol G | | (3) | 2.0 |
| | Luvitol EHO | | (4) | 6.0 |
| | Oxynex K flüssig | (Art.-Nr. 108324) | (1) | 0.05 |
| | | | | |
| B | Panthenol | (Art.-Nr. 501375) | (1) | 0.5 |
| | Karion F flüssig | (Art.-Nr. 102993) | (1) | 4.0 |
| | Konservierungsmittel | | | q.s. |
| | Wasser, demineralisiert | | | ad 100 |
| | | | | |
| C | Sepigel 305 | | (5) | 3.0 |
| | | | | |
| D | Ectoin | | (1) | 1.0 |

Als Konservierungsmittel können
0.05 % Propyl-4-hydroxybenzoat (Art.-Nr. 107427) oder
0.15 % Methyl-4-hydroxybenzoat (Art.-Nr. 106757)
verwendet werden.

### Herstellung:

Die vereinigte Phase B wird unter Rühren langsam in die Phase C eingetragen. Danach wird die vorgelöste Phase A zugesetzt. Es wird gerührt bis die Phasen homogen gemischt sind. Anschließend wird Phase D zugegeben und bis zur Homogenität gerührt.

### Bezugsquellen:

(1) Merck KGaA, Darmstadt
(2) Gustav Heess, Stuttgart
(3) Henkel KGaA, Düsseldorf
(4) BASF AG, Ludwigshafen
(5) Seppic, Frankreich

### Beispiel 2

Aus folgenden Komponenten wird eine erfindungsgemäße Creme (O/W) enthaltend Ectoin hergestellt:

| | | | | Gew.-% |
|---|---|---|---|---|
| A | Paraffin, dünnflüssig | (Art.-Nr. 107174) | (1) | 8.0 |
| | Isopropylmyristat | (Art.-Nr. 822102) | (1) | 4.0 |
| | Mirasil CM 5 | | (2) | 3.0 |
| | Stearinsäure | | (1) | 3.0 |
| | Arlacel 165 | | (3) | 5.0 |
| | | | | |
| B | Glycerin, 87 % | (Art.-Nr. 104091) | (1) | 3.0 |
| | Germaben II | | (4) | 0.5 |
| | Wasser, demineralisiert | | | ad 100 |
| | | | | |
| C | Parfüm Bianca | | (5) | 0.3 |
| | | | | |
| D | Ectoin | | (1) | 1.0 |

### Herstellung:

Zunächst werden die Phasen A und B getrennt auf 75 °C erwärmt. Danach wird Phase A unter Rühren langsam zu Phase B gegeben und solange gerührt bis eine homogene Mischung entsteht. Nach Homogenisierung der Emulsion wird unter Rühren auf 30 °C abgekühlt, die Phasen C und D zugegeben und bis zur Homogenität gerührt.

### Bezugsquellen:

(1) Merck KGaA, Darmstadt
(2) Rhodia
(3) ICI
(4) ISP
(5) Dragoco

### Beispiel 3

Aus folgenden Komponenten wird eine erfindungsgemäße Sonnenschutzlotion (W/O) enthaltend Ectoin und den UV-Filter Eusolex T-2000 hergestellt:

| | | | | Gew.-% |
|---|---|---|---|---|
| A | Abil WE 09 | | (2) | 5.0 |
| | Jojoba Öl | | (3) | 6.0 |
| | Cetiol V | | (4) | 6.0 |
| | Prisorine 2021 | | (5) | 4.5 |
| | Ricinusöl | | (6) | 1.0 |
| | Lunacera M | | (7) | 1.8 |
| | Miglyol 812 Neutralöl | | (8) | 4.5 |
| | | | | |
| B | Eusolex T-2000 | (Art.-Nr. 105373) | (1) | 3.0 |
| | Glycerin, 87 % | (Art.-Nr. 104091) | (1) | 2.0 |
| | Natriumchlorid | (Art.-Nr. 106400) | (1) | 0.4 |
| | Konservierungsmittel | | | q.s. |
| | Wasser, demineralisiert | | | ad 100 |
| | | | | |
| C | Parfüm | | (5) | 0.3 |
| | | | | |
| D | Ectoin | | (1) | 1.0 |

Als Konservierungsmittel können
0.05 % Propyl-4-hydroxybenzoat (Art.-Nr. 107427) oder
0.15 % Methyl-4-hydroxybenzoat (Art.-Nr. 106757)
verwendet werden.

### Herstellung:

Zunächst wird Eusolex T-2000 in Phase B eingerührt und auf 80 °C erwärmt. Danach wird Phase A auf 75 °C erwärmt und unter Rühren Phase B langsam zugegeben. Es wird bis zur Homogenität gerührt und anschließend unter Rühren auf 30 °C abgekühlt. Danach werden die Phasen C und D zugegeben und bis zur Homogenität gerührt.

### Bezugsquellen:

(1) Merck KGaA, Darmstadt
(2) Th. Goldschmidt AG, Essen
(3) H. Lamotte, Bremen
(4) Henkel KGaA, Düsseldorf
(5) Unichema, Emmerich
(6) Gustav Heess, Stuttgart
(7) H.B. Fuller, Lüneburg
(8) Hüls Troisdorf AG, Witten

### Beispiel 4

Es wird untersucht, ob Ectoin eine inhibierende Wirkung auf die UVinduzierte Bildung von "Sunburn cells" in organotypischen Hautäuivalenten aufweist.

Die Untersuchungen werden nach der in A.R. Young, The sunburn cell', Photodermatology 4 (1987) 127-134 beschriebenen Methode durchgeführt. Als Zellen werden organotypische Hautmodelle verwendet. Es werden Skinethic® Hautäquivalente der Firma Skinethic, Nice, France (Lot.-Nr. PK 2 DT 07 99 022A 0603), eingesetzt. Ectoin wird unmittelbar vor Beginn der Prüfung eingewogen und in der Einsatzkonzentration von 4 % in EBSS (Firma Gibco BRL; Best.-Nr. 15015-044) gelöst. Die organotypischen Hautäquivalente (Skinethic®) werden je nach Behandlungsgruppe für 24 Stunden mit oder ohne Ectoin (4%) bei 37 °C und 5 % CO₂ inkubiert. Anschließend erfolgt bei beiden Behandlungsgruppen eine UV-Bestrahlung (0, 30, 60, 100, 200, 300 mJ/cm² UVB) mit dem Sonnensimulator (SOL 500, Dr. Hönle). Nach einer weiteren 24-stündigen Inkubation bei 37 °C und 5% CO₂ werden alle Hautäquivalente histologisch aufbereitet und auf induzierte "Sunbum cells" hin untersucht.

Die Ergebnisse sind in den Tabellen 1 und 2 aufgeführt und in Abb. 1 graphisch dargestellt.

**Tab. 1**

| Anzahl ausgezählter "Sunbum cells" in den sechs einzelnen Schnittsektionen der UV-bestrahlten Hautäquivalente (keine Vorbehandlung mit Ectoin). | | | | | | |
|---|---|---|---|---|---|---|
| | Bestrahlung [mJ/cm²] | | | | | |
| Schnitt | 0 | 30 | 60 | 100 | 200 | 300 |
| 1 | 0 | 2 | 9 | 75 | >100 | >100 |
| 2 | 0 | 2 | 14 | 89 | >100 | >100 |
| 3 | 0 | 4 | 16 | 83 | >100 | >100 |
| 4 | 1 | 2 | 12 | 73 | >100 | >100 |
| 5 | 0 | 5 | 14 | 82 | >100 | >100 |
| 6 | 0 | 4 | 18 | 98 | >100 | >100 |
| MW | 0,17 | 3,17 | 13,83 | 83,33 | >100 | >100 |
| SD | 0,41 | 1,33 | 3,13 | 9,22 | 0,00 | 0,00 |
| MW: Mittelwert, SD: Standardabweichung. | | | | | | |

**Tab. 2**

| Anzahl ausgezählter "Sunburn cells" in den sechs einzelnen Schnittsektionen der UV-bestrahlten Hautäquivalente (Vorbehandlung mit Ectoin). | | | | | | |
|---|---|---|---|---|---|---|
| | Bestrahlung [mJ/cm²] | | | | | |
| Schnitt | 0 | 30 | 60 | 100 | 200 | 300 |
| 1 | 0 | 0 | 3 | 8 | 32 | >100 |
| 2 | 0 | 2 | 3 | 9 | 34 | >100 |
| 3 | 0 | 1 | 2 | 9 | 39 | >100 |
| 4 | 0 | 1 | 4 | 10 | 32 | >100 |
| 5 | 0 | 2 | 3 | 10 | 39 | >100 |
| 6 | 0 | 3 | 2 | 8 | 35 | >100 |
| MW | 0,00 | 1,50 | 2,83 | 9,00 | 35,17 | >100 |
| SD | 0,00 | 1,05 | 0,75 | 0,89 | 3,19 | 0,00 |
| MW: Mittelwert, SD: Standardabweichung. | | | | | | |

In Abb. 1 sind die Mittelwerte MW der histologisch ausgewerteten Schnitte sowie die Standardabweichung SD aus Tab. 1 und Tab. 2 aufgeführt.

Die histologische Betrachtung der beiden unbestrahlten Kontroll-Kulturen (ohne und mit 24-stündiger Vorinkubation mit Ectoin) zeigt, daß es sich um ein lebendes, mehrsschichtiges Epithel handelt. An den Hautäquivalenten war deutlich zu erkennen, daß diesem Epithel ein gut strukturiertes Stratum corneum als äußerste Barriere aufliegt. Zwischen Stratum comeum und den noch vitalen Zellschichten sind deutlich die stark granulierten Zellen des Stratum granulosum Äquivalentes zu erkennen. In sämtlichen analysierten Schnittsektionen der unbestrahlten Kontrollen war in tieferen Schichten nur eine diskeratotische Zelle nachweisbar.

Die epidermalen Gewebe, welche mit 30 mJ/cm² UVB bestrahlt wurden, weisen grundsätzlich den gleichen morphologischen Aufbau auf. Es sind jedoch bereits vereinzelt Sunburn cells (SBCs) nachweisbar, wobei tendenziell im Präparat ohne Vorkonditionierung mehr SBCs nachweisbar sind (vgl. Tab.2).

Nach der Bestrahlung mit 60 mJ/cm² UVB setzt sich diese Tendenz fort. Es sind nun in den mit Ectoin vorbehandelten organotypischen Hautmodellen zwar vereinzelt SBCs detektierbar (2,83 ± 0,75 pro Sektion), jedoch deutlich weniger als in dem nicht mit Ectoin behandelten Hautmodell (vgl. Tab. 2).

Bei einer Bestrahlungsdosis von 100 mJ/cm² UVB ist nach wie vor bei beiden Versuchsansätzen ohne und mit 24-stündiger Vorinkubation mit Ectoin die Übersichtsmorphologie erhalten geblieben, die Anzahl UVinduzierter SBCs nimmt jedoch im Versuchsansatz ohne Vorbehandlung mit Ectoin drastisch zu (83,33 ± 9,22 pro Sektion), wohingegen es sich im vorbehandelten Modell immer noch um äußerst seltene Ereignisse handelt (9 ± 0,89) (vgl. Tab. 2).

Nach der Bestrahlung mit 200 mJ/cm² UVB kommt es bei dem nicht vorbehandelten Hautmodell bereits zu so massiven Schädigung und SBC Bildung, daß eine genaue Quantifizierung nicht mehr möglich ist. Die mit Ectoin vorbehandite Kultur weist nun auch deutliche Bildungsraten von SBCs auf. Die Anzahl (35,17±3,19) bleibt jedoch deutlich unter dem Niveau des 100 mJ/cm² UVB Ansatzes ohne Vorbehandlung (vgl. Tab. 2).

Nach der Bestrahlung mit 300 mJ/cm² UVB ist bei beiden Versuchsansätzen der UV-vermittelte Schaden so groß, daß eine Quantifizierung gebildeter SBCs nicht mehr möglich ist. Bei dieser gewählten hohen UV-Dosis kommt es bereits teilweise zu nekrotischen Gewebezerstörungen.

Es konnte somit gezeigt werden, daß zwischen der Bildungsrate von SBCs und der verabreichten UV-Dosis eine Dosiswirkungsbeziehung gegeben ist. Diese Dosiswirkungsbeziehung konnte jedoch durch eine Vorbehandlung mit Ectoin signifikant in höhere UV-Dosisbereiche verschoben werden.

## Patentansprüche

1. Verwendung von einer oder mehreren Verbindungen ausgewählt aus den Verbindungen der Formeln la und lb 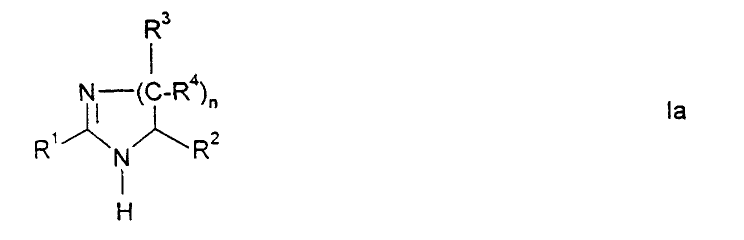 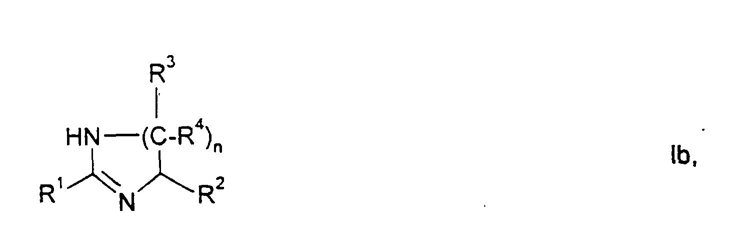 den physiologisch verträglichen Salzen der Verbindungen der Formeln la und lb, und den stereoisomeren Formen der Verbindungen der Formeln la und lb, wobei
R¹ H oder Alkyl,
R² H, COOH, COO-Alkyl oder CO-NH-R⁵,
R³ und R⁴ jeweils unabhängig voneinander H oder OH,
n 1, 2 oder 3,
Alkyl einen Alkylrest mit 1 bis 4 Kohlenstoffatomen, und
R⁵ H, Alkyl, einen Aminosäurerest, Dipeptidrest oder Tripeptidrest
bedeuten zur Herstellung einer Formulierung zum Schutz und zur Stabilisierung der Nukleinsäuren von menschlichen Hautzellen.

2. Verwendung nach Anspruch 1, **dadurch gekennzeichnet, daß** die Nukleinsäuren von menschlichen Hautzellen vor physikalischen, chemischen und biologischen Einflüssen geschützt und stabilisiert werden.

3. Verwendung nach Anspruch 1, **dadurch gekennzeichnet, daß** die Nukleinsäuren von menschlichen Hautzellen vor UV-Strahlung geschützt werden.

4. Verwendung nach Anspruch 1, **dadurch gekennzeichnet, daß** die Nukleinsäuren von menschlichen Hautzellen vor denaturierenden Substanzen geschützt und stabilisiert werden.

5. Verwendung nach Anspruch 1, **dadurch gekennzeichnet, daß** die Nukleinsäuren von menschlichen Hautzellen vor Enzymen geschützt und stabilisiert werden.

6. Verwendung nach Anspruch 1, **dadurch gekennzeichnet, daß** die Nukleinsäuren von menschlichen Hautzellen vor Viren geschützt und stabilisiert werden.

7. Verwendung nach Anspruch 1, **dadurch gekennzeichnet, daß** die Nukleinsäuren von menschlichen Hautzellen vor Herpes-Viren geschützt und stabilisiert werden.

8. Verwendung nach Anspruch 3, **dadurch gekennzeichnet, daß** die Formulierung zusätzlich zu einer oder mehreren Verbindungen ausgewählt aus den Verbindungen der Formeln la und lb, den physiologisch verträglichen Salzen der Verbindungen der Formeln la und lb und den stereoisomeren Formen der Verbindungen der Formeln la und lb einen oder mehrere UV-Filter enthält.

9. Verwendung nach einem der Ansprüche 1 bis 8, **dadurch gekennzeichnet, daß** die Nukleinsäuren der Zellen der Epidermis geschützt und stabilisiert werden.

10. Verwendung nach einem der Ansprüche 1 bis 9, **dadurch gekennzeichnet, daß** man eine oder mehrere Verbindungen ausgewählt aus den Verbindungen der Formeln la und lb, den physiologisch verträglichen Salzen der Verbindungen der Formeln la und lb und den stereoisomeren Formen der Verbindungen der Formeln la und lb gegebenenfalls zusammen mit einem oder mehreren UV-Filtern zur äußeren Anwendung in Form einer Lösung, einer Suspension, einer Emulsion, einer Paste, einer Salbe, eines Gels, einer Creme, einer Lotion, eines Puders, einer Seife, eines tensidhaltigen Reinigungspräparates, eines Öls, eines Lippenstifts, eines Lippenpflegestifts, einer Mascara, eines Eyeliners, von Lidschatten, von Rouge, eines Puder-, Emulsions- oder Wachs-Make ups, eines Sonnenschutz-, Prä-Sun- und After-Sun-Präparats oder eines Sprays einsetzt.

11. Verwendung nach einem der Ansprüche 1 bis 10, **dadurch gekennzeichnet, daß** der Anteil der Verbindungen ausgewählt aus den Verbindungen der Formeln la und lb, den physiologisch verträglichen Salzen der Verbindungen der Formeln la und lb, und den stereoisomeren Formen der Verbindungen der Formeln la und lb von 0,0001 bis 50 Gew.% bezogen auf die gesamte Formulierung beträgt.

12. Verwendung nach einem der Ansprüche 1 bis 11, **dadurch gekennzeichnet, daß** die Verbindungen der Formeln la und lb ausgewählt sind aus den Verbindungen (S)-1,4,5,6-Tetrahydro-2-methyl-4-pyrimidincarbonsäure und (S,S)-1,4,5,6-Tetrahydro-5-hydroxy-2-methyl-4-pyrimidincarbonsäure.

13. Formulierung enthaltend
a) eine oder mehrere Verbindungen ausgewählt aus den Verbindungen der Formeln la und lb 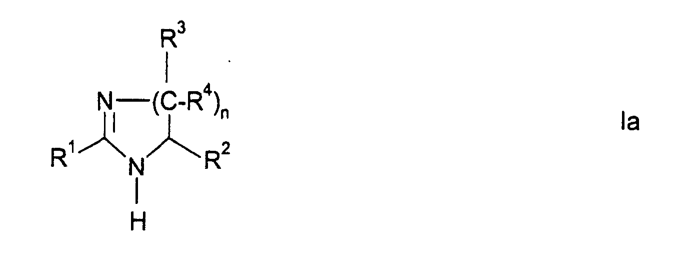 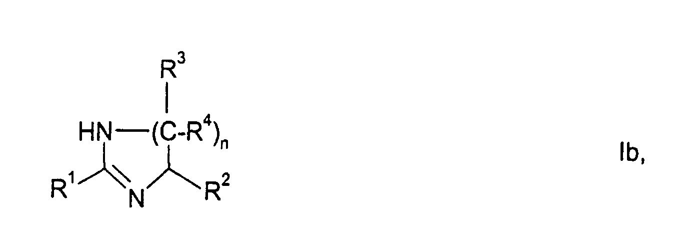 den physiologisch verträglichen Salzen der Verbindungen der Formeln la und lb, und den stereoisomeren Formen der Verbindungen der Formeln la und lb, wobei
R¹ H oder Alkyl,
R² H, COOH, COO-Alkyl oder CO-NH-R⁵,
R³ und R⁴ jeweils unabhängig voneinander H oder OH,
n 1, 2 oder 3,
Alkyl einen Alkylrest mit 1 bis 4 Kohlenstoffatomen, und
R⁵ H, Alkyl, einen Aminosäurerest, Dipeptidrest oder Tripeptidrest
bedeuten
und
b) einen oder mehrere UV-Filter.

14. Formulierung nach Anspruch 13, **dadurch gekennzeichnet, daß** die Verbindungen der Formeln la und lb ausgewählt sind aus den Verbindungen (S)-1,4,5,6-Tetrahydro-2-methyl-4-pyrimidincarbonsäure und (S,S)-1,4,5,6-Tetrahydro-5-hydroxy-2-methyl-4-pyrimidincarbonsäure.

15. Formulierung nach einem der Ansprüche 13 oder 14, **dadurch gekennzeichnet, daß** die UV-Filter ausgewählt sind aus organischen und anorganischen UV-Filtern.

16. Formulierung nach Anspruch 15, **dadurch gekennzeichnet, daß** die organischen UV-Filter ausgewählt sind aus Benzoyl- oder Dibenzoylmethanderivaten, Methoxyzimtsäureestern, Salicylatderivaten, Benzylidencampherderivaten, Octocrylen, Benzophenonen, Phenylbenzimidazol-5-sulfonsäure, 4-Aminobenzoesäure, Octyl-Triazon und Octyl-dimethyl PABA.

17. Formulierung nach einem der Ansprüche 15 oder 16, **dadurch gekennzeichnet, daß** die anorganischen UV-Filter ausgewählt sind aus Titandioxid und Zinkoxid.

18. Formulierung nach einem der Ansprüche 13 bis 17, **dadurch gekennzeichnet, daß** der Anteil der Verbindungen ausgewählt aus den Verbindungen der Formeln la und lb, den physiologisch verträglichen Salzen der Verbindungen der Formeln la und lb, und den stereoisomeren Formen der Verbindungen der Formeln la und lb von 0,0001 bis 50 Gew.% bezogen auf die gesamte Formulierung beträgt und der Anteil der UV-Filter von 0,01 bis 10 Gew.% bezogen auf die gesamte Formulierung beträgt.

19. Formulierung nach einem der Ansprüche 13 bis 18, **dadurch gekennzeichnet, daß** sie in Form einer Lösung, einer Suspension, einer Emulsion, einer Paste, einer Salbe, eines Gels, einer Creme, einer Lotion, eines Puders, einer Seife, eines tensidhaltigen Reinigungspräparates, eines Öls, eines Lippenstifts, eines Lippenpflegestifts, einer Mascara, eines Eyeliners, von Lidschatten, von Rouge, eines Puder-, Emulsions- oder Wachs-Make ups, eines Sonnenschutz-, Prä-Sun- und After-Sun-Präparats oder eines Sprays vorliegt.

## Claims

1. Use of one or more compounds selected from the compounds of the formulae la and lb 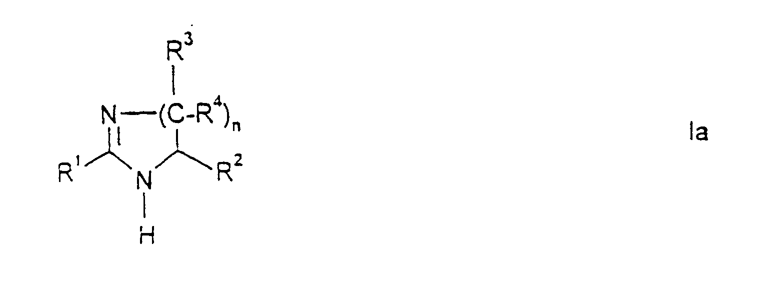 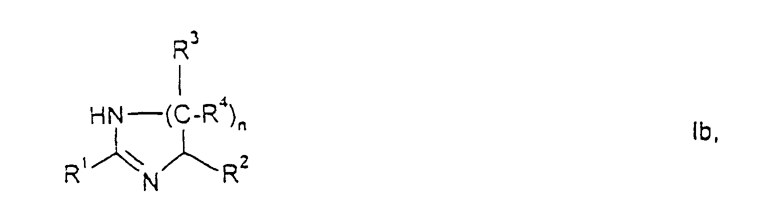 the physiologically tolerated salts of the compounds of the formulae la and lb, and the stereoisomeric forms of the compounds of the formulae la and lb, where
R¹ is H or alkyl,
R² is H, COOH, COO-alkyl or CO-NH-R⁵,
R³ and R⁴ are each, independently of one another, H or OH,
n is 1, 2 or 3,
alkyl is an alkyl radical having from 1 to 4 carbon atoms, and
R⁵ is H, alkyl, an amino acid radical, dipeptide radical or tripeptide radical,
for the preparation of a formulation for the protection and stabilisation of nucleic acids of human skin cells.

2. Use according to Claim 1, **characterised in that** the nucleic acids of human skin cells are protected and stabilised against physical, chemical and biological influences.

3. Use according to Claim 1, **characterised in that** the nucleic acids of human skin cells are protected against UV radiation.

4. Use according to Claim 1, **characterised in that** the nucleic acids of human skin cells are protected and stabilised against denaturing substances.

5. Use according to Claim 1, **characterised in that** the nucleic acids of human skin cells are protected and stabilised against enzymes.

6. Use according to Claim 1, **characterised in that** the nucleic acids of human skin cells are protected and stabilised against viruses.

7. Use according to Claim 1, **characterised in that** the nucleic acids of human skin cells are protected and stabilised against herpes viruses.

8. Use according to Claim 3, **characterised in that** the formulation, in addition to one or more compounds selected from the compounds of the formulae la and lb, the physiologically tolerated salts of the compounds of the formulae la and lb, and the stereoisomeric forms of the compounds of the formulae la and lb, comprises one or more UV filters.

9. Use according to one of Claims 1 to 8, **characterised in that** the nucleic acids of the cells of the epidermis are protected and stabilised.

10. Use according to one of Claims 1 to 9, **characterised in that** one or more compounds selected from the compounds of the formulae la and lb, the physiologically tolerated salts of the compounds of the formulae la and lb, and the stereoisomeric forms of the compounds of the formulae la and lb, if desired together with one or more UV filters, are employed for topical application in the form of a solution, a suspension, an emulsion, a paste, an ointment, a gel, a cream, a lotion, a powder, a soap, a surfactant-containing cleaning preparation, an oil, a lipstick, a lipcare stick, a mascara, an eyeliner, eyeshadow, rouge, powder, emulsion or wax makeup, a sunscreen, presun or aftersun preparation or a spray.

11. Use according to one of Claims 1 to 10, **characterised in that** the proportion of the compounds selected from the compounds of the formulae la and lb, the physiologically tolerated salts of the compounds of the formulae la and lb, and the stereoisomeric forms of the compounds of the formulae la and lb is from 0.0001 to 50% by weight, based on the entire formulation.

12. Use according to one of Claims 1 to 11, **characterised in that** the compounds of the formulae la and lb are selected from the compounds (S)-1,4,5,6-tetrahydro-2-methyl-4-pyrimidinecarboxylic acid and (S,S)-1,4,5,6-tetrahydro-5-hydroxy-2-methyl-4-pyrimidinecarboxylic acid.

13. Formulation comprising
a) one or more compounds selected from the compounds of the formulae la and lb 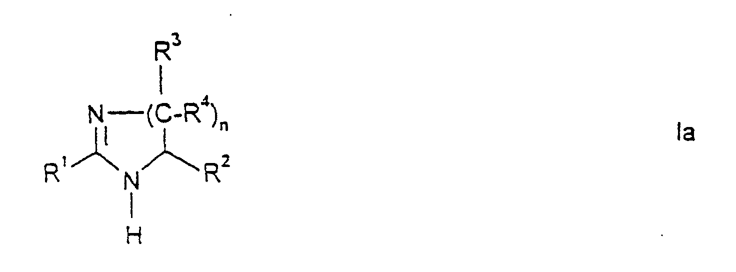 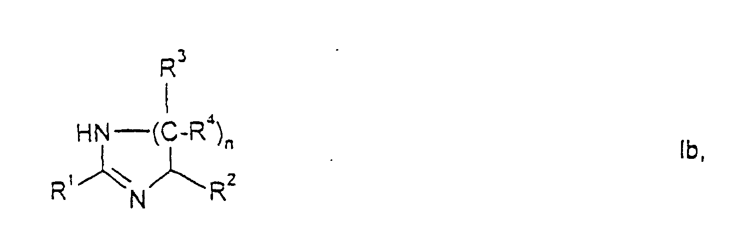 the physiologically tolerated salts of the compounds of the formulae la and lb, and the stereoisomeric forms of the compounds of the formulae la and lb, where
R¹ is H or alkyl,
R² is H, COOH, COO-alkyl or CO-NH-R⁵,
R³ and R⁴ are each, independently of one another, H or OH,
n is 1, 2 or 3,
alkyl is an alkyl radical having from 1 to 4 carbon atoms, and
R⁵ is H, alkyl, an amino acid radical, dipeptide radical or tripeptide radical,
and
b) one or more UV filters.

14. Formulation according to Claim 13, **characterised in that** the compounds of the formulae la and lb are selected from the compounds (S)-1,4,5,6-tetrahydro-2-methyl-4-pyrimidinecarboxylic acid and (S,S)-1,4,5,6-tetrahydro-5-hydroxy-2-methyl-4-pyrimidinecarboxylic acid.

15. Formulation according to one of Claims 13 and 14, **characterised in that** the UV filters are selected from organic and inorganic UV filters.

16. Formulation according to Claim 15, **characterised in that** the organic UV filters are selected from benzoyl- or dibenzoylmethane derivatives, methoxycinnamic acid esters, salicylate derivatives, benzylidenecamphor derivatives, octocrylene, benzophenones, phenylbenzimidazole-5-sulfonic acid, 4-aminobenzoic acid, octyltriazone and octyldimethyl-PABA.

17. Formulation according to one of Claims 15 and 16, **characterised in that** the inorganic UV filters are selected from titanium dioxide and zinc oxide.

18. Formulation according to one of Claims 13 to 17, **characterised in that** the proportion of the compounds selected from the compounds of the formulae la and lb, the physiologically tolerated salts of the compounds of the formulae la and lb, and the stereoisomeric forms of the compounds of the formulae la and lb is from 0.0001 to 50% by weight, based on the entire formulation, and the proportion of the UV filters is from 0.01 to 10% by weight, based on the entire formulation.

19. Formulation according to one of Claims 13 to 18, **characterised in that** it is in the form of a solution, a suspension, an emulsion, a paste, an ointment, a gel, a cream, a lotion, a powder, a soap, a surfactant-containing cleaning preparation, an oil, a lipstick, a lipcare stick, a mascara, an eyeliner, eyeshadow, rouge, powder, emulsion or wax makeup, a sunscreen, presun or aftersun preparation or a spray.

## Revendications

1. Utilisation d'un ou plusieurs composés choisis parmi les composés de formules Ia et Ib 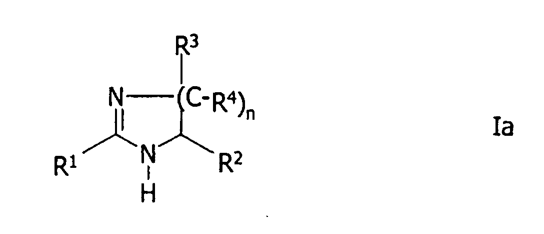 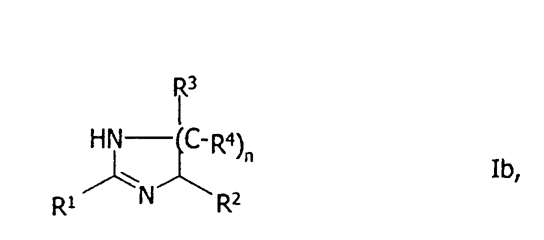 les sels physiologiquement compatibles des composés de formules Ia et Ib et les formes stéréoisomères des composés de formules Ia et Ib où
R¹ représente H ou un alkyle,
R² H, COOH, COO-alkyle ou CO-NH-R⁵,
R³ et R⁴ représentent, indépendamment l'un de l'autre, H ou OH,
n est égal à 1, 2 ou 3,
l'alkyle représente un reste alkyle comportant 1 à 4 atomes de carbone et
R⁵ H, un alkyle, un reste acide aminé, un reste dipeptide ou un reste tripeptide,
pour la préparation d'une formulation destinée à protéger et à stabiliser les acides nucléiques des cellules de peau humaines.

2. Utilisation selon la revendication 1, **caractérisée en ce que** les acides nucléiques des cellules de peau humaines sont protégés des influences physiques, chimiques et biologiques et sont stabilisés.

3. Utilisation selon la revendication 1, **caractérisée en ce que** les acides nucléiques des cellules de peau humaines sont protégés du rayonnement UV.

4. Utilisation selon la revendication 1, **caractérisée en ce que** les acides nucléiques des cellules de peau humaines sont protégés des substances dénaturantes et sont stabilisés.

5. Utilisation selon la revendication 1, **caractérisée en ce que** les acides nucléiques des cellules de peau humaines sont protégés des enzymes et sont stabilisés.

6. Utilisation selon la revendication 1, **caractérisée en ce que** les acides nucléiques des cellules de peau humaines sont protégés des virus et sont stabilisés.

7. Utilisation selon la revendication 1, **caractérisée en ce que** les acides nucléiques des cellules de peau humaines sont protégés des virus de l'herpès et sont stabilisés.

8. Utilisation selon la revendication 3, **caractérisée en ce que** la formulation contient, en plus d'un ou plusieurs composés choisis parmi les composés de formules Ia et Ib, les sels physiologiquement compatibles des composés de formules Ia et Ib et les formes stéréoisomères des composés de formules Ia et Ib, un ou plusieurs filtres UV.

9. Utilisation selon l'une des revendications 1 à 8, **caractérisée en ce que** les acides nucléiques des cellules de l'épiderme sont protégés et stabilisés.

10. Utilisation selon l'une des revendications 1 à 9, **caractérisée en ce que** l'on utilise un ou plusieurs composés choisis parmi les composés de formules Ia et Ib, les sels physiologiquement compatibles des composés de formules Ia et Ib et les formes stéréoisomères des composés de formules Ia et Ib, éventuellement associés à un ou plusieurs filtres UV, pour un usage externe sous la forme d'une solution, d'une suspension, d'une émulsion, d'une pâte, d'un onguent, d'un gel, d'une crème, d'une lotion, d'une poudre, d'un savon, d'une préparation nettoyante contenant un agent tensio-actif, d'une huile, d'un bâton de rouge à lèvres, d'un stick de soin pour lèvres, d'un mascara, d'un eyeliner, d'une ombre pour paupières, de fard, d'un maquillage sous forme de poudre, d'émulsion ou de cire, d'une préparation antisolaire, présolaire et postsolaire ou d'un spray.

11. Utilisation selon l'une des revendications 1 à 10, **caractérisée en ce que** la proportion des composés choisis parmi les composés de formules Ia et Ib, les sels physiologiquement compatibles des composés de formules Ia et Ib et les formes stéréoisomères des composés de formules Ia et Ib est comprise entre 0,0001 et 50 % en poids rapporté à la formation totale.

12. Utilisation selon l'une des revendications 1 à 11, **caractérisée en ce que** les composés de formules Ia et Ib sont choisis parmi les composés l'acide (S)-1,4,5,6-tétrahydro-2-méthyl-4-pyrimidine carboxylique et l'acide (S,S)-1,4,5,6-tétrahydro-5-hydroxy-2-méthyl-4-pyrimidine carboxylique.

13. Formulation contenant
a) un ou plusieurs composés choisis parmi les composés de formules Ia et Ib 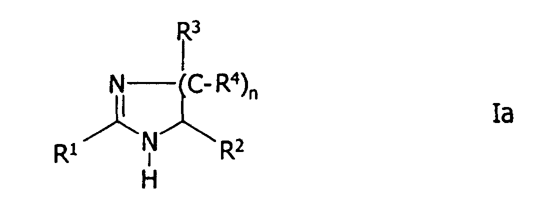 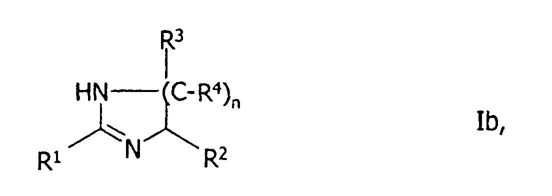 les sels physiologiquement compatibles des composés de formules Ia et Ib et les formes stéréoisomères des composés de formules Ia et Ib, où
R¹ représente H ou un alkyle,
R² H, COOH, COO-alkyle ou CO-NH-R⁵,
R³ et R⁴ représentent, indépendamment l'un de l'autre, H ou OH,
n est égal à 1, 2 ou 3,
l'alkyle représente un reste alkyle comportant 1 à 4 atomes de carbone et
R⁵ H, un alkyle, un reste acide aminé, un reste dipeptide ou un reste tripeptide
et
b) un ou plusieurs filtres UV.

14. Formulation selon la revendication 13, **caractérisée en ce que** les composés de formules Ia et Ib sont choisis parmi les composés l'acide (S)-1,4,5,6-tétrahydro-2-méthyk-4-pyrimidine carboxylique et l'acide (S,S)-1,4,5,6-tétrahydro-5-hydroxy-2-méthyl-4-pyrimidine carboxylique.

15. Formulation selon l'une des revendications 13 et 14, **caractérisée en ce que** les filtres UV sont choisis parmi les filtres UV organiques et inorganiques.

16. Formulation selon la revendication 15, **caractérisée en ce que** les filtres UV organiques sont choisis parmi les dérivés du benzoylméthane ou du dibenzoylméthane, les esters de l'acide méthoxycinnamique, les dérivés de salicylate, les dérivés du benzylidène camphre, les octocryles, les benzophénones, l'acide phénylbenzimidazol-5-sulfonique, l'acide 4-aminobenzoïque, l'octyltriazone et l'octyldiméthylP.A.B.A.

17. Formulation selon l'une des revendications 15 et 16, **caractérisée en ce que** les filtres UV inorganiques sont choisis parmi les dioxyde de titane et l'oxyde de zinc.

18. Formulation selon l'une des revendications 13 à 17, **caractérisée en ce que** la proportion des composés choisis parmi les composés de formules Ia et Ib, les sels physiologiquement compatibles des composés de formules Ia et Ib et les formes stéréoisomères des composés de formules Ia et Ib est comprise entre 0,0001 et 50 % en poids rapporté à la formulation totale et la proportion des filtres UV est comprise entre 0,01 et 10 % en poids rapporté à la formulation totale.

19. Formulation selon l'une des revendications 13 à 18, **caractérisée en ce qu'**elle se présente sous la forme d'une solution, d'une suspension, d'une émulsion, d'une pâte, d'un onguent, d'un gel, d'une crème, d'une lotion, d'une poudre, d'un savon, d'une préparation nettoyante contenant un agent tensioactif, d'une huile, d'un bâton de rouge à lèvres, d'un stick de soin pour lèvres, d'un mascara, d'un eyeliner, d'une ombre pour paupières, de fard, d'un maquillage sous forme de poudre, d'émulsion ou de cire, d'une préparation antisolaire, présolaire ou postsolaire ou d'un spray.
